# EUROPEAN PATENT APPLICATION

(11) **EP 4 238 574 A1**
(43) Date of publication of application: **06.09.2023**
(21) Application number: 21886613.5
(22) Date of filing: 12.10.2021
(51) Int. Cl.: A61K 38/17, A61P 21/00, A61P 25/00, A23L 33/17, G01N 33/50, G01N 33/68

(54) **COMPOSITION FOR PREVENTION OR TREATMENT OF NEUROMUSCULAR DISEASES COMPRISING CDO PROTEIN OR GENE ENCODING SAME**

(30) Priority: 27.10.2020 KR 20200140438
(71) Applicant: Animuscure Inc., Suwon-si, Gyeonggi-do 16419 (KR)
(72) Inventor: KANG, Jong Sun, Suwon-si, Gyeonggi-do 16418 (KR); AN, Su Bin, Suwon-si, Gyeonggi-do 16362 (KR); BAE, Ju Hyeon, Suwon-si, Gyeonggi-do 16421 (KR); KIM, Hye Been, Suwon-si, Gyeonggi-do 16362 (KR)
(74) Representative: Nederlandsch Octrooibureau
(86) International application number: PCT/KR2021/013970
(87) International publication number: WO 2022/092619

(57) **Abstract**

The present invention relates to a use of a cell adhesion moleculerelated/down-regulated by oncogene (Cdo) protein or a gene encoding the same for preventing or treating neuromuscular diseases caused by damage to motor neurons, particularly muscle stem cells and neuromuscular junctions, due to aging, oxidative stress, and the like; and a method for screening for candidates for activating the expression of Cdo. When Cdo is deleted in motor neurons or neuromuscular junctions, aggravation of damage to degenerative motor neurons due to aging, and DNA damage caused by oxidative stress and inflammation are caused, which may cause neuromuscular diseases. Thus, the diseases can be treated through overexpression and activity of the Cdo protein and the gene encoding the same.

## Description

### [Technical Field]

The present invention relates to a use of a Cdo protein expressed in motor neurons and neuromuscular junctions, and a gene encoding the same for preventing or treating neuromuscular diseases.

### [Background Art]

Skeletal muscle is primarily regenerated by muscle stem cells called satellite cells (SCs). Satellite cells are normally in a quiescent state, and are activated by stimuli such as injury to promote self-renewal and differentiation. After satellite cells differentiate, they return to a quiescent state, which is important for maintaining stem cell populations.

Various muscle wasting conditions associated with aging or malnutrition are associated with decreases in the number and proliferative capacity of satellite cells as described above. Satellite cells progressively lose their ability to proliferate and differentiate with aging. The aging and dysfunction of satellite cells may accumulate DNA damage and arrest the cell cycle to induce cellular senescence. Aged cells have characteristic features such as a flat appearance, senescence-associated-β-galactosidase (SA-β-gal) activity, and upregulation of cyclin-dependent kinase inhibitors p16Ink4a (p16) and p21Waf1 (p21). Further, aged cells produce the senescence-associated secretory phenotype (SASP), producing inflammatory cytokines and insulin-like growth factor-binding proteins. Although the dysregulation of cell cycle inhibitors is known to be the critical mechanism underlying cellular senescence in the related art, the molecular mechanisms of satellite cellular senescence have not yet been studied much.

A cell surface protein Cdo (also referred to as Cdon) (Homo sapiens Gene ID: 50937; Mus musculus Gene ID: 57810) is a cell surface receptor of the immunoglobulin/fibronectin type III repeat family involved in muscle differentiation. Cdo forms complexes with N-cadherin at contact sites between skeletal myoblasts, and such interactions are known to have important effects on the promyogenic function of Cdo. However, the mechanisms at various motor neurons or neuromuscular junctions in which Cdo is expressed are not known.

A neuromuscular disease is a disease that occurs in the peripheral nerves and muscles, and a peripheral nervous system disease includes a disease of the peripheral nerve itself, dysfunction of the peripheral nerves caused by damage to the peripheral nerves due to a disease of the surrounding tissue adjacent to the peripheral nerves, and diseases which occur in neuromuscular junctions where the peripheral nerves are connected to the muscles, and muscles themselves.

In addition, a motor neuron disease induced by abnormalities in the peripheral nervous system as described above refers to a neurological disease in which muscle strength is normal, but motor neurons that control the activity thereof are damaged due to degenerative progression, and is caused by various factors such as genetic factors, environmental factors, toxin poisoning, viral infections, complications of other diseases, and aging. With the aging of the population, the prevalence of degenerative motor neuron diseases, particularly caused by aging, is increasing, which may have a significant impact on human quality of life, so that there is a need for research on the fundamental cause and treatment method.

### [Disclosure]

### [Technical Problem]

Thus, while studying various proteins and genes associated with damage to motor neurons, the present inventors found that, by performing research on a cell adhesion molecule-related/down-regulated by oncogene (Cdo) protein expressed in muscle stem cells, it is possible to prevent damage to motor neurons due to cellular stress caused by various factors through the expression regulation of the protein and a gene encoding the same, and achieve the effects of treating and alleviating a disease caused by damage to motor neurons, thereby completing the present invention.

Therefore, an object of the present invention is to provide a composition for protecting motor neuron cells, comprising a Cdo protein or a gene encoding the same.

Another object of the present invention is to provide a composition for preventing, ameliorating or treating a neuromuscular disease, comprising a Cdo protein or a gene encoding the same.

Still another object of the present invention is to provide a method for screening a material for preventing, ameliorating or treating a neuromuscular disease in relation to whether Cdo is expressed.

### [Technical Solution]

To achieve the objects described above, the present invention provides a composition for protecting motor neuron cells, comprising a Cdo protein or a gene encoding the same.

The present invention also provides a method for protecting motor neuron cells, the method comprising: administering a Cdo protein or a gene encoding the same to a subject in need thereof.

The present invention also provides a use of a Cdo protein or a gene encoding the same for protecting motor neuron cells.

The present invention also provides a use of a Cdo protein or a gene encoding the same for preparing a drug for protecting motor neuron cells.

Furthermore, to achieve other objects of the present invention, the present invention provides a composition for preventing, ameliorating or treating a neuromuscular disease, comprising a Cdo protein or a gene encoding the same.

The present invention also provides a method for preventing, ameliorating or treating a neuromuscular disease, the method comprising: administering a Cdo protein or a gene encoding the same to a subject in need thereof.

The present invention also provides a use of a Cdo protein or a gene encoding the same for preventing, ameliorating or treating a neuromuscular disease.

The present invention also provides a use of a Cdo protein or a gene encoding the same for preparing a drug for preventing, ameliorating or treating a neuromuscular disease.

As an aspect of the present invention, the composition of the present invention has effects of protecting motor neurons and preventing, ameliorating or treating a neuromuscular disease, and thus, may be used for various purposes, such as a pharmaceutical, a health functional food, a functional food, an animal feed and a cell culture solution composition.

To achieve other objects of the present invention, the present invention provides a method for screening a therapeutic material of a neuromuscular disease, the method comprising: i) treating motor neuron cells or satellite cells with a candidate *in vitro;* ii) determining the expression or activity of Cdo in the motor neuron cells or satellite cells treated with the candidate; and iii) selecting candidates which enhance the expression or activity of Cdo compared to a non-treatment group as candidates for treating a neuromuscular disease.

Hereinafter, the present invention will be described in detail.

The present invention relates to a composition for protecting motor neuron cells, comprising a Cdo protein or a gene encoding the same.

The present invention also relates to a method for protecting motor neuron cells, the method comprising: administering a Cdo protein or a gene encoding the same to a subject in need thereof.

The present invention also relates to a use of a Cdo protein or a gene encoding the same for protecting motor neuron cells.

The present invention also relates to a use of a Cdo protein or a gene encoding the same for preparing a drug for protecting motor neuron cells.

The protection of the motor neuron cells of the present invention particularly includes protecting motor neurons from degenerative damage due to aging, but is not limited to, and is to improve a survival rate, maintain body weight, maintain behavioral ability, repair damaged nerves, repair DNA damage, and protect motor neuron cells from cellular stress, and refers to the alleviation or reduction of stress which may occur in motor neuron cells due to various factors such as aging.

As an aspect of the present invention, the protection of motor neurons is to prevent damage to motor neurons from aging, oxidative stress, and inflammatory stress, prevent muscular atrophy and fibrosis due to the damage to motor neurons, and reduce the stress sensitivity of motor neuron cells, and includes promoting the reinnervation of damaged motor neuron cells.

Further, the present invention relates to a composition for preventing, ameliorating or treating a neuromuscular disease, comprising a Cdo protein or a gene encoding the same.

The present invention also relates to a method for preventing, ameliorating or treating a neuromuscular disease, the method comprising: administering a Cdo protein or a gene encoding the same to a subject in need thereof.

The present invention also relates to a use of a Cdo protein or a gene encoding the same for preventing, ameliorating or treating a neuromuscular disease.

The present invention also relates to a use of a Cdo protein or a gene encoding the same for preparing a drug for preventing, ameliorating or treating a neuromuscular disease.

The neuromuscular disease is caused by abnormalities in the peripheral nervous system, and includes muscular dystrophy, a motor neuron disease, myopathy, a neuromuscular junction disease, and the like as a neurological disease in which the motor neurons that control muscle movements are damaged due to degenerative progression. In addition, although not limited thereto, the neuromuscular diseases are classified as diseases of the neuromuscular junctions and muscles (disease classification code G70-G73), and includes myasthenia gravis, muscle nerve disorders, muscular dystrophy, myotonic disorders, myopathy, primary muscle disorders, myasthenic syndrome, Lambert-Eaton syndrome, and the like. Furthermore, the motor neuron disease includes amyotrophic lateral sclerosis (ALS), infantile progressive spinal muscular atrophy (SMA), progressive bulbar palsy, pseudobulbar palsy, progressive muscular atrophy (PMA), progressive lateral sclerosis (PLS), monomelic amyotrophy (MMA), Huntington's disease, or the like, and includes other motor neuron diseases, and the like.

The present inventors confirmed that the expression of Cdo in motor neurons, satellite cells which are muscle stem cells, or neuromuscular junctions was reduced by stress stimulus such as aging, and particularly confirmed that an animal model in which Cdo was specifically decreased in motor neurons showed a significant decrease in survival rate and motor ability. Through this, it can be seen that the presence or absence of Cdo expression in motor neurons may affect various diseases associated with motor neurons. Further, it could be confirmed that the number of nuclei in the neuromuscular junction decreased when Cdo specifically decreased in satellite cells. These results indicate that Cdo plays a role in determining the type of muscle fiber, and in particular, it was confirmed that Cdo may also affect single muscle fiber differentiation.

As an aspect of the present invention, the composition of the present invention may be used for various purposes such as a pharmaceutical, a health functional food, a functional food, an animal feed and a cell culture solution composition, and has an effect of preventing, ameliorating or treating a neuromuscular disease by preventing damage to motor neuron cells.

As used herein, the term "prevention" refers to all actions that suppress a neuromuscular disease or delay the onset of the neuromuscular disease by administering the pharmaceutical composition according to the present invention.

As used herein, the term "treatment" refers to all actions that ameliorate or beneficially change symptoms of a neuromuscular disease by administering the pharmaceutical composition according to the present invention.

As used herein, the term "subject" may refer to all animals including humans, in need of the motor neuron protection according to the present invention, or who have developed or are likely to develop a neuromuscular disease. The animal may be not only a human but also a mammal such as a cow, a horse, a sheep, a pig, a goat, a camel, an antelope, a dog, and a cat in need of treatment of similar symptoms.

The pharmaceutical composition according to the present invention may be used by being formulated in the form of an oral formulation, such as a powder, a granule, a pill, a capsule, a suspension, an emulsion, a syrup, and an aerosol, an external preparation, a suppository, and a sterile injection solution, according to a typical method, and may further include a carrier, an excipient, or the like required for formulation. Examples of pharmaceutically acceptable carriers, excipients or diluents which may be further included in the pharmaceutical composition include lactose, dextrose, sucrose, sorbitol, mannitol, xylitol, erythritol, maltitol, starch, acacia rubber, alginate, gelatin, calcium phosphate, calcium silicate, cellulose, methyl cellulose, microcrystalline cellulose, polyvinyl pyrrolidone, water, methyl hydroxybenzoate, propyl hydroxybenzoate, talc, magnesium stearate, mineral oil, and the like. When the pharmaceutical composition is formulated, the pharmaceutical composition is prepared using a diluent or excipient, such as a filler, an extender, a binder, a wetting agent, a disintegrant, and a surfactant, which are commonly used.

For example, a solid formulation for oral administration includes a tablet, a pill, a powder, a granule, a capsule, and the like, and the solid formulation is prepared by mixing at least one excipient, for example, starch, calcium carbonate, sucrose or lactose, gelatin, and the like with the extract or compound. Further, in addition to a simple excipient, lubricants such as magnesium stearate and talc are also used. A liquid formulation for oral administration corresponds to a suspension, a liquid for internal use, an emulsion, a syrup, and the like, and the liquid formulation may include, in addition to water and liquid paraffin which are simple commonly used diluents, various excipients, for example, a wetting agent, a sweetener, a fragrance, a preservative, and the like.

Examples of a formulation for parenteral administration include an aqueous sterile solution, a non-aqueous solvent, a suspension, an emulsion, a freeze-dried preparation, and a suppository. As the non-aqueous solvent and the suspension, it is possible to use propylene glycol, polyethylene glycol, a vegetable oil such as olive oil, an injectable ester such as ethyl oleate, and the like. As a base of the suppository, it is possible to use Witepsol, Macrogol, Tween 61, cacao butter, laurin fat, glycerogelatin, and the like.

The pharmaceutical composition of the present invention may be orally administered or may be parenterally administered (applied intravenously, subcutaneously, intraperitoneally, or locally), and the administration dose may vary depending on a patient's condition and body weight, severity of disease, drug form, and administration route and period according to the target method, and the administration dose may be selected in an appropriate form by those skilled in the art.

The pharmaceutical composition of the present invention is administered in a pharmaceutically effective amount. In the present invention, the "pharmaceutically effective amount" refers to an amount sufficient to treat diseases as an amount applicable to medical treatment, and the criteria thereof may be determined according to types of diseases of patients, the severity of disease, the activity of drugs, sensitivity to drugs, administration time, administration route, excretion rate, treatment period, ingredients used in combination, and other items. The pharmaceutical composition of the present invention may be administered as an individual therapeutic agent or may be administered in combination with other therapeutic agents, and may be administered sequentially or simultaneously with therapeutic agents in the related art. The administration dose may be determined at a level that can minimize side effects in consideration of all of the above factors, and may be easily determined by those skilled in the art. Specifically, the administration dose of the pharmaceutical composition may vary depending on the patient's age, body weight, severity, sex, and the like, and generally, 0.001 to 150 mg of the pharmaceutical composition and more preferably, 0.01 to 100 mg of the pharmaceutical composition, per 1kg of the body weight, may be administered daily or every other day, or one to three times a day. However, this is exemplary, and the admiration dose may be set differently, if necessary.

In addition, the composition of the present invention may be a food or health functional food, and in particular, the "health functional food" refers to a food manufactured and processed using raw materials or ingredients that have functional properties that are useful for the human body according to Health Functional Food Act No. 6727, and "functional" is meant to be taken for the purpose of regulating nutrients to the structure and function of the human body, or obtaining effects useful for public health use, such as physiological effects.

The food or health functional food of the present invention can be manufactured and processed in a pharmaceutical administration form such as a powder, a granule, a tablet, a capsule, a pill, a suspension, an emulsion, and a syrup, or as a health functional food such as a tea bag, an infusion, a beverage, a candy, a jelly, and a gum.

The food or health functional food composition of the present invention may be used as a food additive, and may be commercialized alone or in combination with other ingredients. Furthermore, the food or health functional food composition of the present invention may include nutrients, vitamins, electrolytes, flavors, colorants, enhancers, pectic acids and salts thereof, alginic acid and salts thereof, organic acids, protective colloid thickeners, pH adjusters, stabilizers, preservatives, glycerin, alcohols, carbonating agents used in carbonated drinks, and the like. The ingredients may be used alone or in combination, and may be used in combination in suitable amounts.

Further, the present invention provides a method for screening a therapeutic material of a motor neuron disease, the method comprising: i) treating motor neuron cells or satellite cells with a candidate *in vitro;* ii) determining the expression or activity of Cdo in the motor neuron cells or satellite cells treated with the candidate; and iii) selecting candidates which enhance the expression or activity of Cdo compared to a non-treatment group as candidates for treating a motor neuron disease.

In the present invention, the determining of the expression or activity of Cdo may be performed by various methods capable of measuring the amount of RNA or protein produced by the expression of the Cdo gene. Preferably, the expression level of RNA was confirmed using real-time PCR (RT-PCR), a microarray, northern blotting, and the like, and protein activity and expression may be confirmed by a method such as immunoprecipitation, immunostaining, chromatin immunostaining, enzyme-linked immunosorbent assay (ELISA), and western blotting, and is not limited to that method.

### [Advantageous Effects]

The composition of the present invention includes a Cdo protein or a gene encoding the same, and the Cdo protein is a factor that plays an important role associated with the prevention of damage to motor neuron cells, and has effects of preventing damage to motor neurons from stress due to various factors, and preventing, ameliorating and treating motor neuron diseases, and the like due to the damage to motor neurons and degenerative factors.

### [Description of Drawings]

FIG. 1 shows the results of confirming the survival rate of mice specifically lacking Cdo in motor neurons.
FIG. 2 illustrates the results of footprint evaluation to observe changes in motor ability in mice specifically lacking Cdo in motor neurons.
FIG. 3 shows the results of behavioral changes confirmed by an open field test and a muscle strength test in mice specifically lacking Cdo in motor neurons.
FIGS. 4 and 5 shows the results of confirming the decrease in the expression level of Cdo in aged mice.
FIG. 6 shows the results of observing changes in neuromuscular junctions of mice specifically lacking Cdo in muscle stem cells (satellite cells).
FIG. 7 shows the results of observing changes in the expression levels of nuclear envelope structural genes (Syne1, Syne2) in mice specifically lacking Cdo in muscle stem cells (satellite cells).

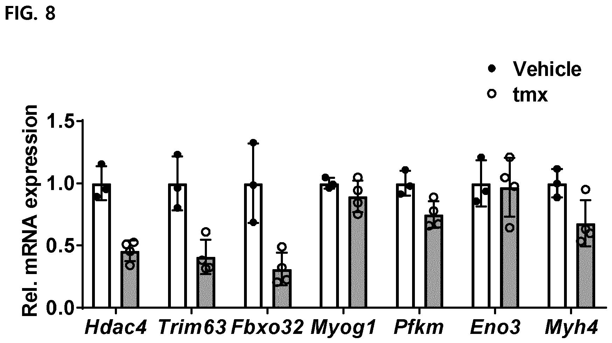

FIG. 8 shows the results of confirming the gene expression levels of mice specifically lacking Cdo in muscle stem cells (satellite cells).
FIG. 9 confirms the expression of Cdo in the neuromuscular junction of a single muscle fiber.
FIG. 10 shows the results (co-staining) of confirming both the expression of Cdo and a motor neuron marker (ChAT) in mouse spinal cords.
FIG. 11 shows the results of staining the neuromuscular junctions of mice specifically lacking Cdo in motor neurons using a motor neuron axon marker (NF) and an activity marker (synaptophysin).
FIGS. 12 and 13 illustrate mice specifically lacking Cdo in muscle stem cells (satellite cells), and changes in Cdo expression in the neuromuscular junctions of single muscle fibers.
FIG. 14 shows the results of *in vitro* confirmation of the types of genes expressed from Cdo-deficient muscle stem cells (satellite cells).

### [Modes of the Invention]

Hereinafter, the present specification will be described in detail with reference to Examples in order to specifically explain the present specification. However, the Examples according to the present specification may be modified in various forms, and it is not interpreted that the scope of the present specification is limited to the Examples described below in detail. The Examples of the present specification are provided to more completely describe the present specification to a person with ordinary skill in the art.

### Example 1. Materials and experimental methods

### 1-1) Construction of experimental models

Constant conditions (temperature 22 ± 2°C, humidity 55 ± 5%) were maintained in an experimental animal breeding room. A light cycle of 12 hours a day and a dark cycle of 12 hours a day were adapted.

Mice specifically lacking Cdo in motor neurons were constructed by crossing mice carrying a Cdo-floxed allelic gene (Cdo f/f purchased from EUCOMM) with transgenic mice expressing a Cre recombinant enzyme under the control of an Hb9 promoter (Hb9-Cre provided by Jackson Laboratory). For mice specifically lacking Cdo in muscle stem cells, Cdo ^{fl/fl};Pax7 ^{ERT2Cre+/-} mice were constructed by crossing mice carrying a Cdo-floxed alleic gene (Cdo f/f purchased from EUCOMM) with transgenic mice expressing a Cre recombinant enzyme under the control of a Pax7 promoter (Pax7 ERT2Cre provided by Jackson Laboratory). In particular, the muscle stem cell-specific Cdo deficiency was induced by injecting tamoxifen (100 mg/kg body weight) intraperitoneally into Cdo ^{fl/fl};Pax7 ^{ERT2Cre+/-} mice 5 times every 2 days.

For aged mice (Geriatric model), wild-type mice, which had not been transformed, were maintained in a breeding facility for 27 months. As a control group, wild-type 5-month-old mice were used.

### 1-2) Behavior analysis

To analyze differences in behavior between Cdo f/f and Cdo hb9cre mice, an Open field test, a Gait test and a Grip test were performed.

The Open field test was performed in an open plastic container (30 × 30 × 30 cm). When each experiment was initiated, mice were placed in the corners of the plastic container and movements were recorded for 20 minutes. Noldus EthoVision 13.0 tracking software was used to analyze the distance traveled and speed.

The Gait test was performed by a method of placing an A4 sheet on the bottom of a treadmill apparatus (Columbus Exer-6 M treadmill) with transparent walls and allowing the mouse to walk straight in one direction. After the hind paws of the mouse were sufficiently painted with purple ink, the stride, sway, and stance of the mouse footprints recorded on the A4 paper were measured.

The Grip test was measured using a test apparatus (Bioseb). When the front paw of the mouse fully grasped the grid of the apparatus, the mouse was pulled with a constant force until the front paw fell off the grid, and the force measured at this time was recorded.

The test of each mouse was repeated three times and was conducted as a blind test.

### 1-3) Cell culture and transfection

C2C12 cells were cultured in a composition of 15% FBS, 10 units/mL penicillin, and 10 µg/mL streptomycin based on DMEM media.

To make Cdo expression deficient in C2C12 cells, cells were infected with Lentiviral pLKO. 1-puro, Cdo-shRNA.

### 1-4) Protein and RNA analysis

After cells and tissues were harvested, cells were lysed by adding a lysis buffer (20 mM Tris-HCl, pH8, 150 mM NaCl, 1% Triton X, proteinase inhibitor) for 30 minutes, then centrifuged at 13000 rpm for 30 minutes, and then a cell lysate sample was quantified, the same amount of protein was subjected to SDS-PAGE electrophoresis, and transferred to a PVDF membrane. After reaction with the corresponding primary and secondary antibodies, protein expression levels were confirmed by exposing the protein to X-ray film using an ECL reagent.

C2C12 cells and muscle tissue were homogenized with FastPrepR-24 (MP Biomedicals) and RNA was extracted using an easy-spin Total RNA Extraction Kit (iNtRON Biotechnology). Fold changes in gene expression were normalized compared to the expression of the ribosomal gene L32.

### 1-5) Histology and immunofluorescence analysis

The tibialis anterior muscle, extensor digitorum longus muscle, and lumbar spine were used as tissues used in the experiments, and the tibialis anterior muscle and lumbar spine were frozen and fixed with an OCT compound using liquefied nitrogen immediately after isolation, and then stored in an ultra-low temperature freezer at -80°C. Tissue sections were cut into thicknesses of 10 um and 7 um using a cryomicrotome maintained at -20°C.

Immunohistochemical staining was performed using the tibialis anterior muscle and lumbar spine isolated from the animal tissues. The frozen sections stored in an ultra-low temperature freezer at -80°C were washed twice with 1×PBS. The frozen sections were fixed at ordinary temperature for 15 minutes using 4% paraformaldehyde and washed twice with 1xPBS. The tissue was reacted with a permeabilization buffer (0.5% Triton-X/PBS) at ordinary temperature for 5 minutes, and washed twice with 1xPBS. The tissue was reacted with a TE buffer at 100°C for 10 minutes for antigen retrieval, and washed twice with 1×PBS. After blocking with a blocking buffer (5% goat serum, 0.1% Triton-X) for 1 hour, a primary antibody (neurofilament, synaptophysin) was diluted 1:300 in the blocking buffer and reacted at 4°C for 24 hours, and then washed three times with 1xPBS. A secondary antibody and BTX antibody conjugated with the secondary antibody were reacted at room temperature for 2 hours, and then washed twice with 1×PBS. Thereafter, they were encapsulated and analyzed under a confocal fluorescence microscope (LSM710).

Neuromuscular junction staining (NMJ staining) was performed using the extensor digitorum longus muscle isolated from the animal tissue. The extensor digitorum longus muscle was isolated, immediately fixed using 4% paraformaldehyde at ordinary temperature for 15 minutes, and washed three times with 1xPBS. After blocking with a blocking buffer (3% BSA, 0.5% Triton-X) for 2 hours, a primary antibody (neurofilament, synaptophysin) was diluted 1:300 in the blocking buffer and reacted at 4°C for 24 hours, and then washed three times with 1xPBS. A secondary antibody and BTX antibody conjugated with the secondary antibody were reacted at room temperature for 2 hours, and then washed twice with 1xPBS. They were later encapsulated and analyzed under a confocal fluorescence microscope (LSM710).

The mouse extensor digitorum longus (EDL) was used for immunohistochemical staining through single muscle fiber isolation. Each single muscle fiber was isolated from mice with or without induced muscle injury, and muscle injury was induced by intramuscularly injecting 10 µM cardiotoxin diluted in PBS. After EDL muscle isolation from mice, single muscle fiber isolation was performed by treatment with 200 U/ml of collagenase II at 37°C for 90 min, and subsequently, tissue immunochemistry of each single muscle fiber was performed using BTX and Cdo antibodies (1:300). Thereafter, the expression of Cdo in neuromuscular junctions was observed under a confocal fluorescence microscope (LSM710).

### 1-6) Statistical analysis

All values were expressed as mean ± SEM or SD. Statistical significance was calculated by a paired or unpaired two-tailed Student's t-test or analysis of variance (ANOVA) test followed by Tukey's test. Differences were considered significant at P<0.05.

Experimental Example 1. Effect of motor neuron-specific Cdo deficiency

### 1-1) Effect of decrease in survival rate of Cdo-deficient mice

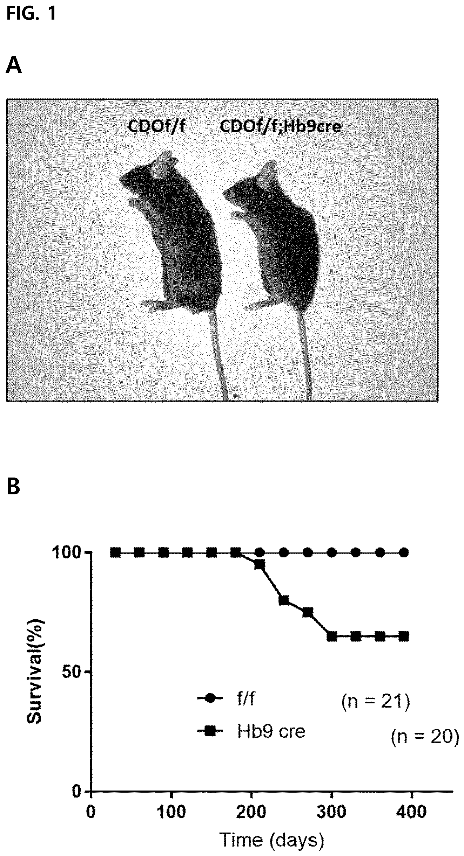

The survival rate and body weight of mice specifically lacking Cdo in motor neurons (Cdo ^{Hb9cre} mice) were measured at each month of age. As a result, it was confirmed that the survival rate of mice decreased rapidly as aging progressed compared to wild type (Cdo ^{f/f}) (FIG. 1). That is, it was confirmed that Cdo, which is expressed in motor neurons, plays an important role in the maintenance of survival rate and body weight of aged mice.

### 1-2) Changes in behavioral ability due to aging in Cdo-deficient mice

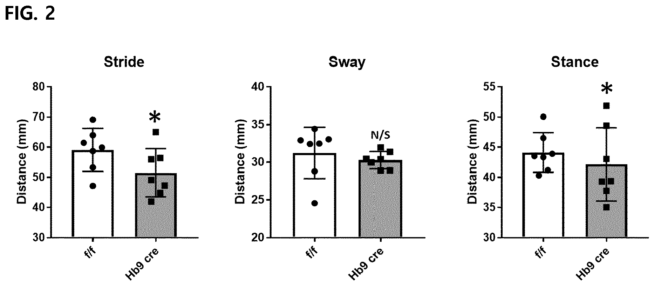

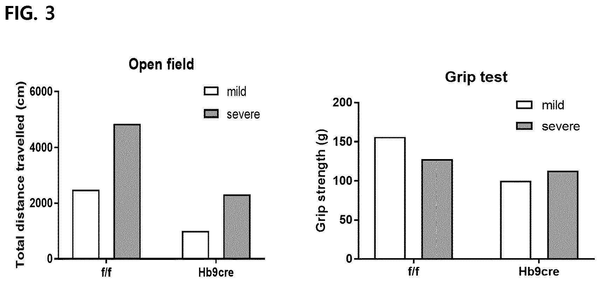

In the case of mice specifically lacking Cdo in motor neurons (Cdo ^{Hb9cre} mice), as can be seen in FIG. 2, it could be confirmed that in the case of Hb9cre in footprint evaluation, motor neurons deteriorated because problems in walking were observed, such as a shorter stride compared to the wild type. In addition, as a result of confirming by performing an open field test as a behavioral evaluation, it was confirmed that the moving distance and walking speed of the Cdo ^{Hb9cre} mice were decreased compared to normal mice (Cdo ^{f/f}) in both the survival group and the death group. Furthermore, as a result of performing a muscle strength evaluation, it was confirmed that muscle strength in both the survival group and the death group of Cdo ^{Hb9cre} mice was decreased compared to the normal control (FIG. 3).

From the experimental results described above, it can be seen that Cdo plays an important role in maintaining behavioral ability in aged mice in motor neurons.

### 1-3) Confirmation of Cdo expression levels of aged mice

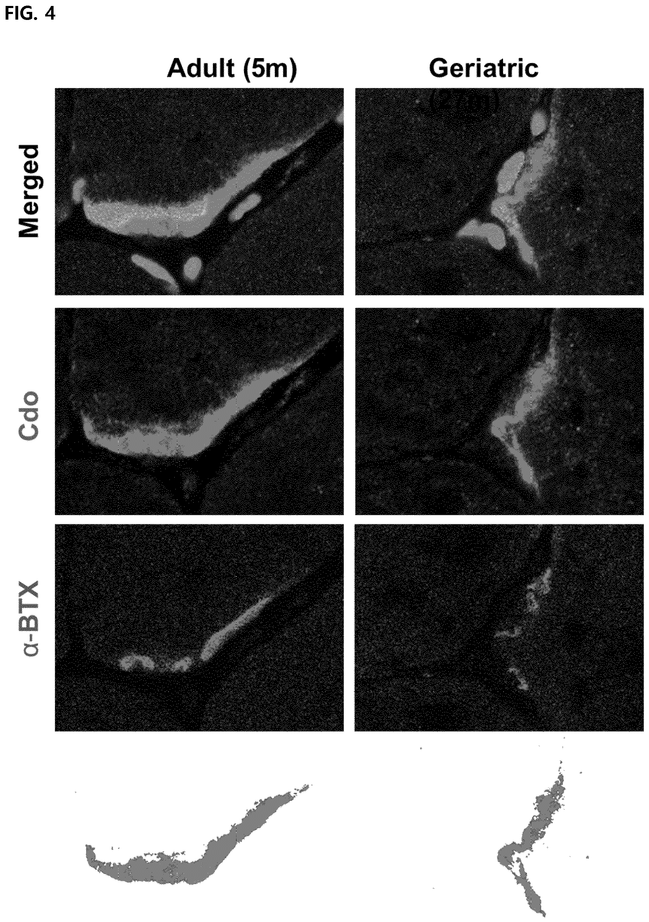

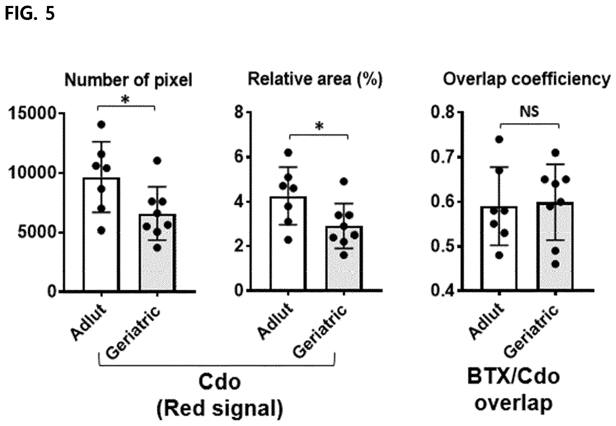

As a result of confirming the expression level of alpha-bungarotoxin (α-BTX), which is a neuromuscular junction marker, in 5-week-old mice and 27-week-old mice, respectively, in order to confirm whether Cdo expression is affected by aging, it was confirmed that the expression of Cdo was reduced (FIGS. 4 and 5).

That is, it was confirmed that Cdo expression was reduced by aging, and the expression was specifically reduced particularly in neuromuscular junctions.

Experimental Example 2. Effects of Cdo deficiency in muscle stem cells (satellite cells)

### 2-1) Changes around neuromuscular junctions in Cdo-deficient mice

Transgenic mice capable of specifically making Cdo deficient in muscle stem cells were used by injecting tamoxifen. After Cdo was specifically depleted in muscle stems, cardiotoxin (CTX), which is a type of snake venom, was injected into the tibialis anterior (TA) muscle and extensor digitorum longus (EDL) muscle to cause damage to muscles in order to observe a phenomenon that occurs during the regeneration process, and then muscles that had completed regeneration were analyzed 2 months later.

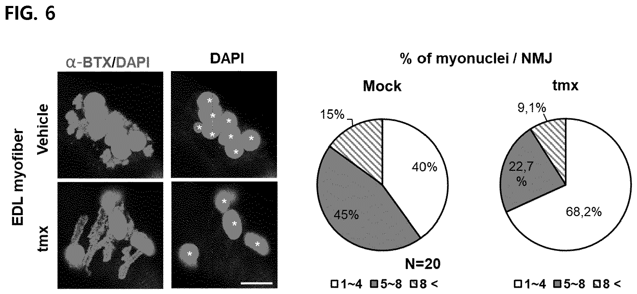

As a result, as illustrated in FIG. 6, as a result of analyzing the neuromuscular junctions in single muscle fibers isolated from the EDL muscle, it was confirmed that the number of nuclei was reduced in the NMJ of mice into which tamoxifen was injected (n = 20; Vehicle, n = 22; tmx).

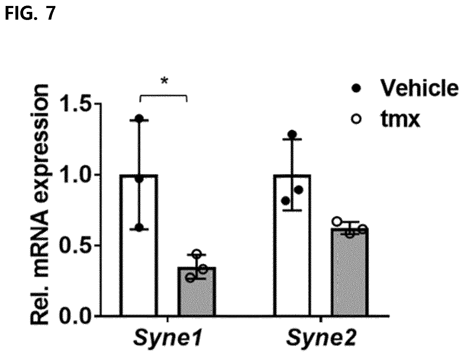

In addition, as illustrated in FIG. 7, it could be confirmed that the mRNA expression of Syn2, which is a nuclear envelope structural protein gene, was significantly reduced in the Cdo-deficient group (TA muscle, n = 3; Vehicle, n = 4; tmx).

In particular, the expression of Hdac4, which is a gene whose expression is increased in glycolytic muscle fibers, was significantly decreased, and accordingly, it was confirmed that the target gene of Hdac4 was generally decreased. In particular, a decrease in the expression of Myh4 (MyHC-IIB), which is the target gene of Hdac4, was confirmed. This result suggests that Cdo is likely to play a role in determining muscle fiber types (TA muscle, n = 3; Vehicle, n = 4; tmx). In particular, it was confirmed that a decrease in the number of nuclei in the neuromuscular junctions that appeared in the tmx group is likely to be characterized according to the muscle fiber type.

In other words, the above experimental results indicate that although the model specifically lacking Cdo in motor neurons did not show a large difference in the formation of the neuromuscular junctions, it is likely to exhibit that the NMJ may be characterized according to the muscle fiber type. Sarcopenia, which is a muscular atrophy disease, or a metabolic disease such as diabetes induces a muscular atrophy phenotype, and one of the most prominent features is the reduction of MyHC-IIb, which is a glycolic muscle fiber. That is, Cdo plays a role in determining muscle fiber type, suggesting the possibility that Cdo is a therapeutic protein for the amyotrophic phenotype.

### 2-2) Confirmation of Cdo expression in single muscle fiber neuromuscular junctions

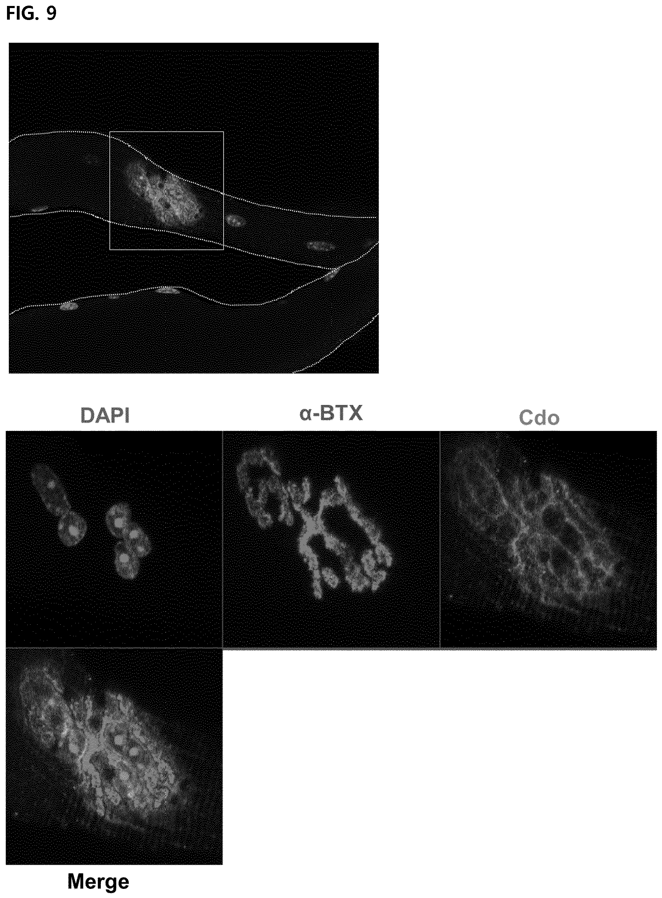

To confirm the specific expression site of Cdo, Cdo and α-BTX, which is a neuromuscular junction marker, were identified by fluorescence staining by isolating single muscle fibers from the EDL muscles of wild-type mice that had not been genetically manipulated. As a result, as illustrated in FIG. 9, it was confirmed that the expression sites of the neuromuscular junction marker and Cdo were almost identical, indicating that Cdo was specifically expressed in the neuromuscular junctions.

### 2-3) Confirmation of Cdo expression in motor neurons

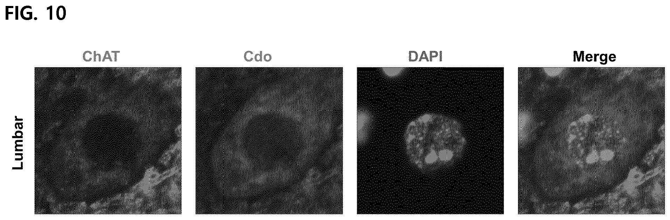

In order to confirm the presence or absence of Cdo expression in motor neurons, Cdo and choline acetyltransferase (ChAT), which is a motor neuron marker, were identified by fluorescence staining in the spinal lumbar spine site of wild-type mice. As a result, as illustrated in FIG. 10, it was confirmed that the expression sites of the motor neuron marker and Cdo almost coincided. Therefore, it can be seen that Cdo is expressed in motor neurons.

### 2-4) Confirmation of neuromuscular junction abnormalities in mice specifically lacking Cdo in motor neurons

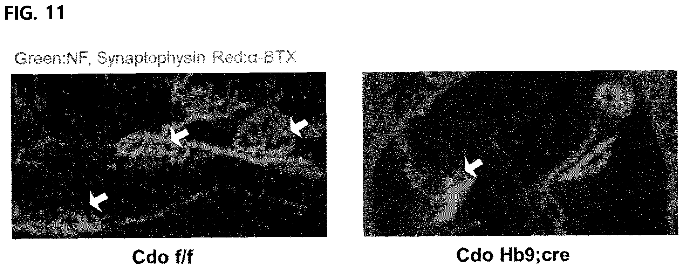

Neurofilament (NF), which is used as a marker for a motor neuron exon, and synaptophysin and α-BTX which are markers that stain a material that aids activity in the neuromuscular junctions in the EDL muscle of mice specifically lacking Cdo in motor neurons, were identified by fluorescence staining. As illustrated in FIG. 11, it was observed that the neuromuscular junctions of mice specifically lacking Cdo in motor neurons failed to maintain their shape (fragmentation) and were formed at relatively thin axons compared to mice which were not Cdo-deficient.

Further, as indicated by arrows in FIG. 11, it could be confirmed that, by observing that synaptophysin and co-staining were reduced, the activity in the neuromuscular junctions was also reduced.

### 2-5) Changes in Cdo expression in mice specifically lacking Cdo in muscle stem cells and single muscle fiber neuromuscular junctions

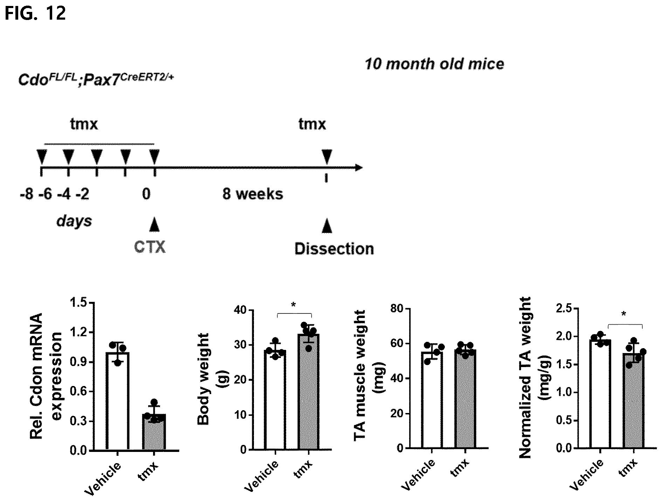

A Cdo ^{fl/fl}; Pax7 ^{ERT2Cre+/-} mouse is a mouse model in which tamoxifen can be injected to specifically remove Cdo in muscle stem cells (satellite cells). As in the schematic view presented in FIG. 12, after Cdo was depleted by injecting tamoxifen (tmx) into mice, CTX damage to the TA-EDL muscle was caused, and then the TA and EDL muscles were isolated and analyzed two months later. Thereafter, the body weight, muscle weight, and muscle weight/body weight values of tamoxifeninjected mice (experimental group) and vehicle (control) were each compared (n = 4 per each group). As a result, as illustrated in FIG. 12, it was confirmed that the mRNA expression of Cdo was significantly reduced in the TA muscle of tamoxifeninjected mice (n = 3; vehicle, n = 4; tmx).

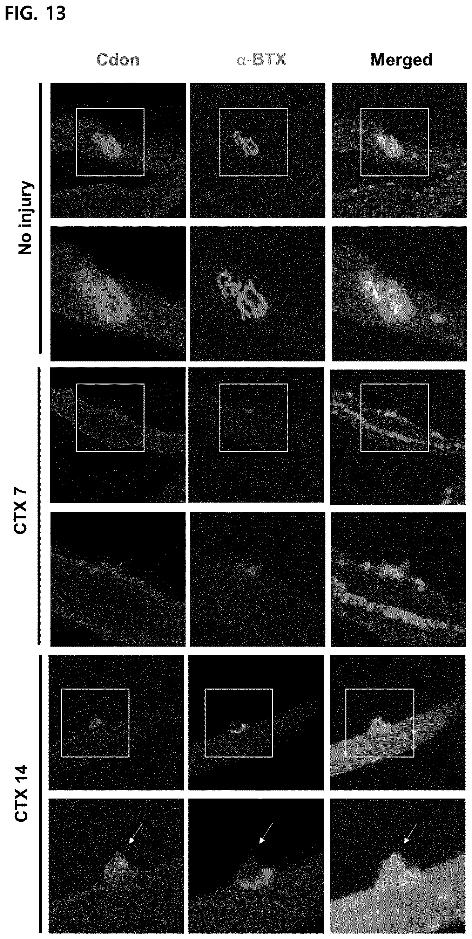

In addition, although Cdo and BTX were expressed in EDL single muscle fibers of wild-type adult mice (5 month-old male), as a result of observation 7 days and 14 days after cardiotoxin (CTX) damage, as illustrated in FIG. 13, it was confirmed that the expression of Cdo was weak in the neuromuscular junctions compared to the muscles of the undamaged group, and the expression of NMJ was observed more clearly 14 days later than on day 7.

From the aforementioned results, it can be seen that the expression of Cdo is important when neuromuscular junctions are formed in damaged muscle fibers.

### 2-6) Changes in muscle type in muscle stem cell-specific Cdo deficiency

By analyzing the mRNA expressed in Cdo-deficient muscle stem cells (C2C12) transfected with an ShCdo-introduced lentivirus, the effect of Cdo deficiency on muscle stem cell differentiation was confirmed.

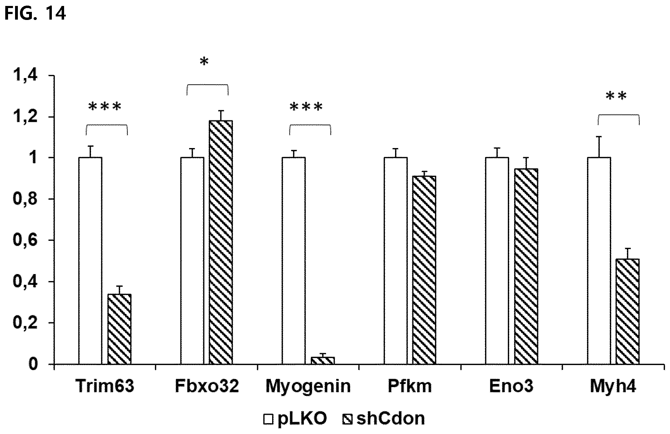

As a result, as illustrated in FIG. 14, it was confirmed that the mRNA expression of Hdac4 and Myh4 was significantly reduced, and it can be seen that this is the same result as *in vivo.*

In the foregoing, the present invention has been examined mainly based on the preferred examples thereof. A person with ordinary skill in the art to which the present invention pertains will be able to understand that the present invention may be implemented in a modified form without departing from the essential characteristics of the present invention. Therefore, the disclosed examples should be considered not from a restrictive viewpoint, but from an explanatory viewpoint. The scope of the present invention is defined not in the above-described explanation, but in the claims, and it should be interpreted that all the differences within a range equivalent thereto are included in the present invention.

## Claims

1. A composition for protecting motor neuron cells, comprising a Cdo protein or a gene encoding the same.

2. The composition of claim 1, wherein the protection of motor neuron cells prevents damage to motor neurons due to aging, oxidative stress and inflammation.

3. The composition of claim 1, wherein the composition promotes the reinnervation of damaged motor neuron cells.

4. A pharmaceutical composition for preventing or treating a neuromuscular disease, comprising a Cdo protein or a gene encoding the same.

5. The composition of claim 4, wherein the neuromuscular disease is selected from the group comprising dystrophy, a motor neuron disease, myopathy, and a neuromuscular junction disease.

6. The composition of claim 5, wherein the motor neuron disease is selected from the group comprising amyotrophic lateral sclerosis (ALS), infantile progressive spinal muscular atrophy (SMA), progressive bulbar palsy, pseudobulbar palsy, progressive muscular atrophy (PMA), progressive lateral sclerosis (PLS), monomelic amyotrophy (MMA) and Huntington's disease.

7. A health functional food composition for preventing or ameliorating a neuromuscular disease, comprising a Cdo protein or a gene encoding the same.

8. The composition of claim 7, wherein the neuromuscular disease is selected from the group comprising dystrophy, a motor neuron disease, myopathy, and a neuromuscular junction disease.

9. The composition of claim 8, wherein the motor neuron disease is selected from the group comprising amyotrophic lateral sclerosis (ALS), infantile progressive spinal muscular atrophy (SMA), progressive bulbar palsy, pseudobulbar palsy, progressive muscular atrophy (PMA), progressive lateral sclerosis (PLS), monomelic amyotrophy (MMA) and Huntington's disease.

10. A method for screening a material of preventing, ameliorating or treating a neuromuscular disease, the method comprising: i) treating motor neuron cells or neuromuscular junctions with a candidate; ii) determining the expression or activity of Cdo in the motor neuron cells or neuromuscular junctions treated with the candidate; and iii) selecting candidates which enhance the expression or activity of Cdo compared to a non-treatment group.
